(19) Europäisches Patentamt

European Patent Office

Office européen des brevets

(11) **EP 1 274 375 B1**

(12) **EUROPÄISCHE PATENTSCHRIFT**

(45) Veröffentlichungstag und Bekanntmachung des Hinweises auf die Patenterteilung:
**08.10.2003 Patentblatt 2003/41**

(51) Int Cl.7: **A61F 2/44**

(21) Anmeldenummer: 01911328.1

(86) Internationale Anmeldenummer:
**PCT/CH01/00179**

(22) Anmeldetag: **21.03.2001**

(87) Internationale Veröffentlichungsnummer:
**WO 01/070144 (27.09.2001 Gazette 2001/39)**

(54) **KÄFIGARTIGES ZWISCHENWIRBELIMPLANTAT**

CAGE-TYPE INTERVERTEBRAL IMPLANT

IMPLANT INTERVERTEBRAL DE TYPE CAGE

(84) Benannte Vertragsstaaten:
**AT BE CH CY DE DK ES FI FR GB GR IE IT LI LU MC NL PT SE TR**

(30) Priorität: **22.03.2000 CH 542002000**

(43) Veröffentlichungstag der Anmeldung:
**15.01.2003 Patentblatt 2003/03**

(73) Patentinhaber: **Scolio GmbH**
**8005 Zürich (CH)**

(72) Erfinder: **MORET, Olivier**
**CH-1950 Sion (CH)**

(74) Vertreter: **Goth, Helmut, Dr.**
**DILTEC AG,**
**Technopark,**
**Technoparkstrasse 1**
**8005 Zürich (CH)**

(56) Entgegenhaltungen:
**EP-A- 0 966 929**       **WO-A-99/65424**
**FR-A- 2 736 537**       **US-A- 5 397 364**

**Beschreibung**

Die Erfindung betrifft ein käfigartiges Zwischenwirbelimplantat sowie ein Verfahren zu dessen Herstellung gemäss den Patentansprüchen 1, bzw. 23.

Es handelt sich um ein Wirbelsäulenimplantat und dessen Herstellungsverfahren, welches zum Einsatz zwischen zwei Wirbelkörper der Wirbelsäule kommt. Es dient als Fusionierung (Arthrodese) der beiden Wirbelkörper und durch sie wird folglich die Original-Bandscheibenhöhe wieder erreicht und auch die Nervenwurzel Foramen geht auf ihre ursprüngliche Grösse zurück.

Die einzelnen Wirbel der Wirbelsäule weisen einen Wirbelkörper, einen Wirbelbogen, einen Dornfortsatz, zwei Querfortsätze und zwei obere und zwei untere Gelenkfortsätze auf. Die Wirbel sind mit den anliegenden Bandscheiben (disci intervertebralis) verbunden und ergeben den Wirbelkörper (corpus vertebrae). Die Bandscheibe besteht aus flüssigkeitsreichem Fasernknorpel und verbindet die einzelnen Wirbelkörper miteinander. Die Grösse der Bandscheiben nimmt von oben nach unten entsprechend der im menschlichen Körper auftretenden Belastungen zu. Die Bandscheiben dienen als elastische Puffer und dämpfen federnd Stösse ab.

Es ist bekannt, dass sich die Bandscheiben belagern können oder dass der innere Gallertkern (nucleus pulposus) durch Risse im bindegewebeartigen, knorpeligen, äusseren Ring (annulus fibrosus) austreten kann. Dabei kann die Bandscheibe teilweise in die Zwischenwirbellöcher (foramina intervertebralia) bzw. in den Spinalkanal eintreten. Ausserdem kann dieser Prolaps dorsal, medial oder lateral sein. Derartige Prolapse treten am häufigsten an den L4-L5-S1 und C6-C7 Vertebrales auf. Werden derartige Prolapse nicht therapiert, kommt es zu irreversiblen Druckschädigung von Nervenwurzeln Foramen oder zu Querschnittsläsionen. Sollten eine symptomatische Physiotherapie, z.B. Krankengymnastik oder Massage, keinen Erfolg versprechen, muss die Bandscheibe (discus intervertebralis) operativ entfernt werden. Nun besteht die Möglichkeit der Implantation eines solchen Implantates (Käfig), wodurch eine Arthrodese zwischen den beiden Wirbelkörpern stattfinden kann.

Aus der EP0916323-A1 ist ein Zwischenwirbelimplantat bekannt, das eine bohnenförmige Struktur aufweist und zwischen den Wirbeln eingesetzt werden kann. das Implantat weist eine Keilform auf, die durch eine unterschiedliche Höhe der beiden Längsseitenwände gegeben ist. Die das Implantat umgebenden Wände sind mit Lochreihen versehen, um die Durchwachsung mit Knochengewebe zu fördern. Nachteilig ist, dass das Implantat lediglich in einer Richtung eine Keilform aufweist und durch die Vielzahl der seitlich angebrachten Löcher aufwendig zu fertigen ist.

Im weiteren sind unter der Bezeichnung 'Brantigan Cage' Käfigstrukturen bekannt, die deren Käfigflächen mit einer Vielzahl von Zähnen belegt sind, um eine unerwünschten Verschiebung des Käfigs zu verhindern. Aus Polyetheretherketon (PEEK) gefertigt, als sog. PEEK-Pressling, weisen sie eine mangelhafte Festigkeit auf, die unter Belastung zum Bruch der Käfigstruktur führen können. Für die Instrumentenaufnahme ist ein einfaches Gewinde vorgesehen, wodurch sich eine ungenügende Instrumentenhandhabung ergibt.
Durch die quaderförmige Geometrie ist ein relativ geringes Kippmoment gegeben, was sich nachteilig auswirkt.

Dokument FR 2 736 537 A1 offenbart ein Zwischenwirbel implantat gemäß des Oberbegriffs von Anspruch 1.

Aufgabe der Erfindung ist es, ein käfigartiges Zwischenwirbelimplantat anzugeben, das eine doppelkeilförmige Geometrie aufweist, die durch zwei Lordosewinkel bedingt ist, und das eine verbesserte Instrumentenhandhabung gewährleistet.
Eine weitere Aufgabe der Erfindung besteht in der Herstellung eines derartigen Implantats.

Erfindungsgemäss wird diese Aufgabe mit einem Implantat gemäss dem Wortlaut nach Patentanspruch 1 und durch ein Verfahren zur Herstellung desselben gemäss dem Wortlaut nach Patentanspruch 23 gelöst.

Die Erfindung wird im Folgenden an Hand von Figuren beschrieben. Es zeigen:

Fig. 1        Perspektivische Darstellung eines Käfigs
Fig. 2        Draufsicht des Käfigs nach Fig. 1
Fig. 3A-3D    Schnittdarstellungen zu Fig. 2
Fig. 4A-4C    Schnittdarstellungen zu Fig. 2
Fig. 5        Schnittdarstellung zu Fig. 2 entlang der abgewickelten Radiuslinie a - a'
Fig. 6A-6B    Seitenansicht des Heckteils mit verschieden angeordneten Führungselementen
Fig. 7        Ausführungsbeispiel für einen Käfig mit einer Zwischenwand und schrägem Heckteil mit Aussparungen in Draufsicht

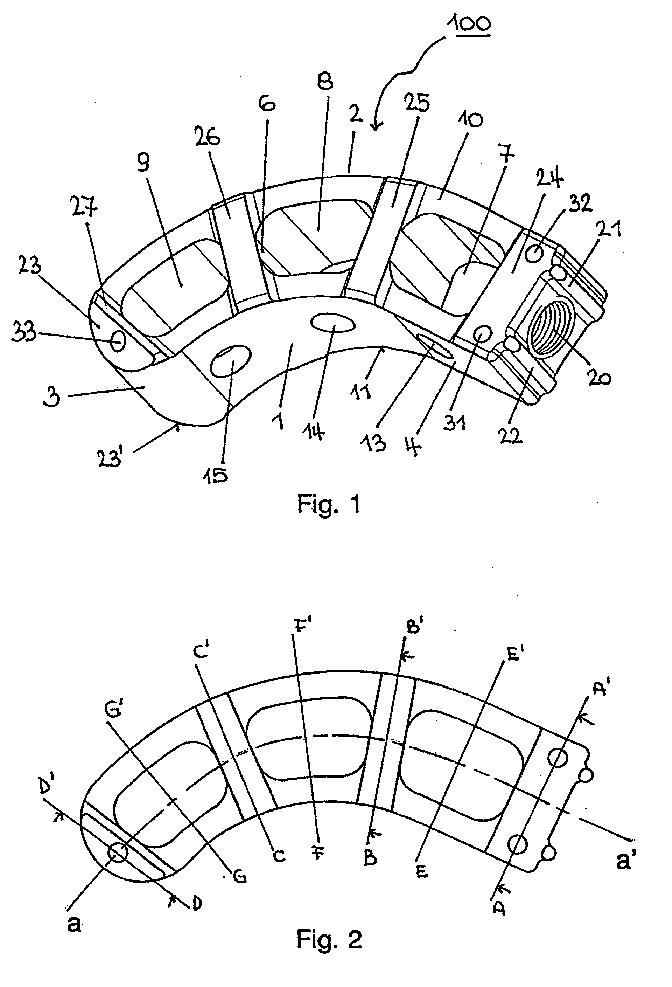

Fig. 1 zeigt in perspektivischer Darstellung einen Käfig 100, der aus einer konkavgekrümmten Seitenwand 1, einer konvex-gekrümmten Seitenwand 2, einem Frontteil 3 und einem Heckteil 4 besteht. Die Seitenwände 1 und 2 sind über Zwischenwände 5 und 6 miteinander verbunden, sodass das Innere des Käfigs in Hohlräume 7, 8 und 9 unterteilt wird. Der inneren Seitenwand 1 ist dabei ein erster innerer Krümmungsradius R1 und der äussere Seitenwand 2 ein zweiter äusserer Krümmungsradius R2 zugeordnet.
Die Seitenwände 1, 2, die Zwischenwände 5, 6, der Frontteil 3 und der Heckteil 4 weisen obere, bzw. untere Begrenzungen auf, die eine obere, bzw. untere Käfigfläche 10, 11 definieren.

Die konkav-gekrümmte Seitenwand 1 weist rund ausgebildete Aussparungen 13, 14 und 15 auf, die in etwa in der Mitte der Hohlräume 7, 8 und 9 angebracht sind und für die Bildung der Knochensubstanz förderlich sind. Die konvex-gekrümmte Seitenwand 2 kann ebenfalls derartige Aussparungen (nicht dargestellt) aufweisen.

Die Käfigflächen 10, 11 weisen im Bereich des Frontteils 3, des Heckteils 4 und der Zwischenwände 5, 6 tafelförmige Überhöhungen 24, 25, 26, 27 auf, die im Wesentlichen parallel zu den Käfigflächen verlaufen, deren Eigenschaften später beschrieben werden.

Der Frontteil 3 ist gerundet ausgebildet und verbindet die Seitenwände 1, 2 des Käfigs über eine gleiche Wandstärke. Er weist frontseitig Abschrägungen 23, 23' auf, die das Einführen und Positionieren des Käfigs im Zwischenwirbelbereich erleichtern.

Der Heckteil 4 ist rechteckig ausgebildet und verbindet die Seitenwände 1, 2 des Käfigs über ebenfalls eine gleiche Wandstärke. Er weist heckseitig eine Bohrung 20 auf, die mit einem Innengewinde versehen ist und für die Instrumentenbefestigung vorgesehen ist. Beidseitig zur Bohrung 20 sind Führungselemente 21 und 22 angeordnet, die hierz.B. als Rippen ausgebildet sind, aber auch aus Aussparungen in Form eines Halbzylinders bestehen können. Die Führungselemente dienen der Instrumentenführung und verhindern beim Lösen des Instrumentes jede unzulässige Drehbewegung des Käfigs. Sobald der Käfig sich in seiner endgültigen Lage zwischen den beiden Wirbeln befindet, was u.a. dann der Fall ist, wenn die Achse des Instrumentes senkrecht zur dorsalen Ebene des Patienten geführt wird, kann das Instrument vom Käfig losgelöst werden. Es hat sich gezeigt, dass sich diese Kontrollmöglichkeit sehr hilfreich und nützlich erwiesen hat.

Der Übergang der Führungselemente, bzw. Rippen 21, 22 zur Fläche des Heckteils 4 ist auf beiden Seiten der Rippe abgerundet, um allfällige Kerbeffekte zu umgehen, was bei Ausführungen aus Kunststoff oder Kompositmaterialien von Bedeutung ist. Im Heckteil 4 und im Frontteil 3 sind Bohrungen 31, 32, resp. 33 für die Aufnahme eines Markers aus einem Metall hoher Dichte vorgesehen sind. Dafür eignen sich besonders Kugeln und/oder Stifte aus Tantal. Die Stifte sind in Bohrungen angeordnet, die entweder senkrecht oder parallel zur Bohrung 20 angeordnet sind. Dadurch kann die Lage des Käfigs während des Eingriffs über einen Bildverstärker beobachtet und beurteilt werden.

Fig. 2 zeigt eine Draufsicht des Käfigs 100 nach Fig. 1 mit Angaben zur Lage der Schnitte A - A' bis D - D' und E - E' bis G - G'. Ebenfalls ersichtlich ist der Verlauf einer Radiuslinie a - a', die durch die Mitte des Käfigs verläuft.

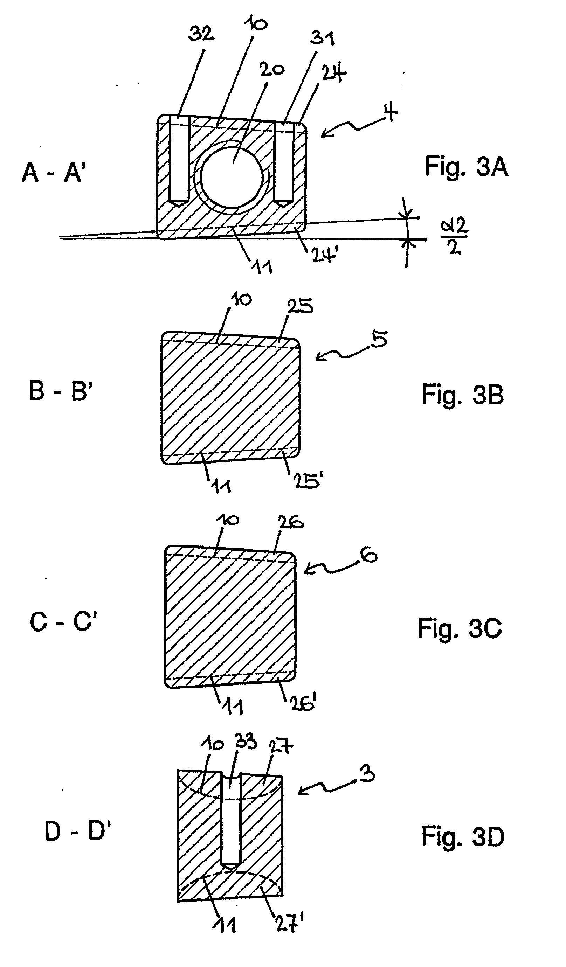

Fig. 3A - 3D zeigen Schnittdarstellungen des Käfigs mit Schnittlagen gemäss Fig. 2. Fig. 3A zeigt einen Schnitt A - A' durch den Heckteil 4. Erkennbar sind die Bohrung 20 für die Instrumentenaufnahme und die Bohrungen 31 und 32 für die Marker.

Im weiteren ist erkennbar, dass die Höhe auf der äusseren Seite des Heckteil grösser ist als die auf der inneren Seite. Damit verläuft die obere Fläche des Heckteils zur unteren - als Teile der Käfigflächen 10 und 11 - nicht mehr parallel. Die beiden Flächen bilden einen Lordosewinkel $\alpha2$, der 0,1 - 4°, vorzugsweise aber 2° beträgt. In Fig. 3A ist dieser Lordosewinkel $\alpha2$ überproportional dargestellt, um besser erkennbar zu sein. Er ist auf der unteren Seite des Heckteils mit $\alpha2/2$ dargestellt.

Falls die Höhe auf der äusseren Seite des Heckteils kleiner ist als die auf der inneren Seite ergibt sich eine entgegengesetzte Schiefe des Käfigs, bzw. eine durch die Käfigflächen 10 und 11 gebildete Keilform, die mit der Keilspitze in die umgekehrte Richtung weist. Wird der Lordosewinkel $\alpha2$ in beiden beschriebenen Fällen identisch null, dann sind die Käfigflächen 10 und 11 als Spezialfall, resp. Grenzfall parallel, was allerdings eine weniger bevorzugte Ausbildung des Käfigs darstellt.

Auf den Teilen der Käfigflächen 10, 11 sind die Überhöhungen 24, 24' angebracht, die hier parallel zu den Käfigflächen ausgebildet sind, dies aber keineswegs zwingend sein müssen.

Fig. 3B und 3C zeigen einen Schnitt B - B', bzw. C - C' durch die Zwischenwände 5, bzw. 6 mit den Überhöhungen 25, 25', bzw. 26, 26', die auf den Teilen der Käfigflächen 10, 11 angebracht sind. Wiederum verlaufen die Überhöhungen parallel zu den Käfigflächen, die den Lordosewinkel $\alpha2$ ebenfalls erkennen lassen.

Fig. 3D zeigt einen Schnitt D - D' durch den Frontteil 3. Erkennbar ist die Bohrung 33 für den Marker. Erkennbar sind die Überhöhungen 27, 27', die auf den Teilen der Käfigflächen 10, 11 des Frontteils 3 angebracht sind. Wiederum verlaufen diese Überhöhungen im Wesentlichen parallel zu den Käfigflächen, die den Lordosewinkel $\alpha2$ ebenfalls erkennen lassen.

Die Überhöhungen 24, 25, 26 und 27, die alle aus den Käfigflächen 10, 11 herausragen, aber lediglich 0,3 - 0,8 mm betragen, dienen der 'Verankerung des Käfigs' nach dem erfolgten Eingriff und helfen eine Migration des Käfigs zu verhindern.

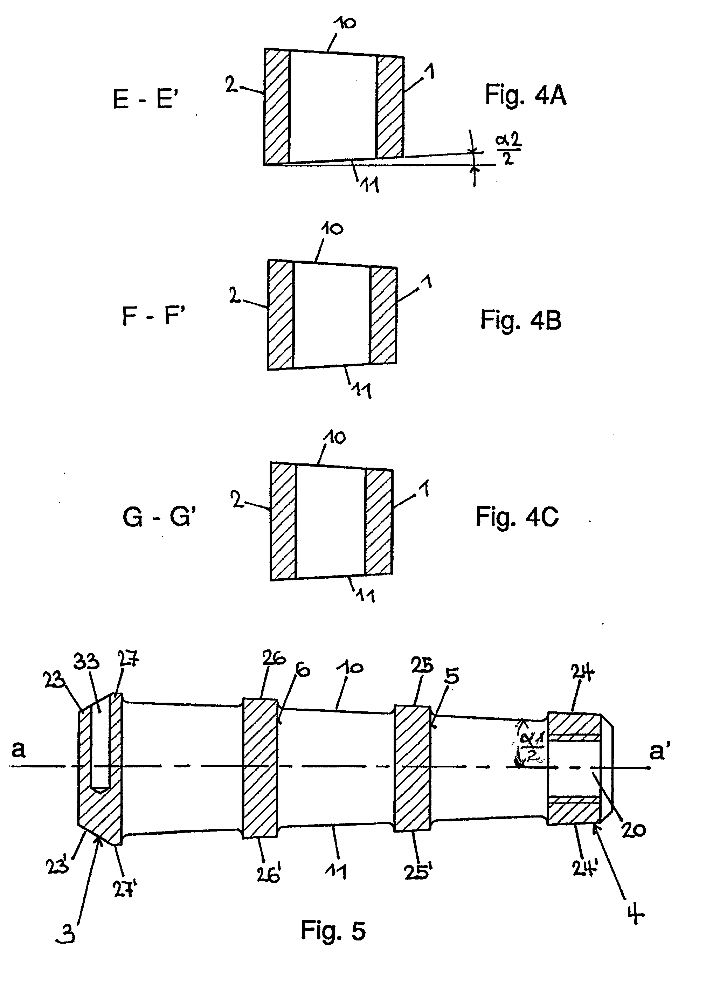

Fig. 4A - 4C zeigen Schnittdarstellungen des Käfigs mit Schnittlagen gemäss Fig. 2. Erkennbar sind die Seitenwände 1, 2, die obere und die untere Käfigfläche 10, 11 und der hälftige Lordosewinkel $\alpha2$, der nur in Fig. 4A einseitig dargestellt ist.

Fig. 5 zeigt eine Schnittdarstellung zu Fig. 2 entlang der abgewickelten Radiuslinie a - a'. Ersichtlich sind im Frontteil 3 die Bohrung 33 für den Marker, die Abschrägungen 23, 23' und die Überhöhungen 27, 27', im Heckteil 4 die Bohrung 20 und die Überhöhungen 24, 24'. Die Zwischenwände 5, 6 weisen die Überhöhungen 25, 25', bzw. 26, 26' auf.

Im weiteren ist erkennbar, dass die Höhe des Frontteils 3 grösser ist als jene des Heckteils 4. Damit verlaufen die Käfigflächen 10 und 11 nicht mehr parallel. Die beiden Flächen bilden einen sogenannten Lordosewinkel $\alpha 1$, der 2 - 8°, vorzugsweise aber 3°, 5° oder 7° beträgt. In Fig. 5 ist diese Nicht-Parallelität bedingt durch den Lordosewinkel $\alpha 1$ überproportional dargestellt, um besser erkennbar zu sein.

Selbstverständlich kann die Käfigstruktur in weiten Grenzen im Rahmen dieser Erfindung abgewandelt werden. So ist beispielsweise die Anzahl der Zwischenwände 5, 6, bzw. jene der Hohlräume 7, 8, 9 nicht auf 2, bzw. 3 beschränkt. Käfigstrukturen mit einer oder mehreren Zwischenwänden sind möglich.

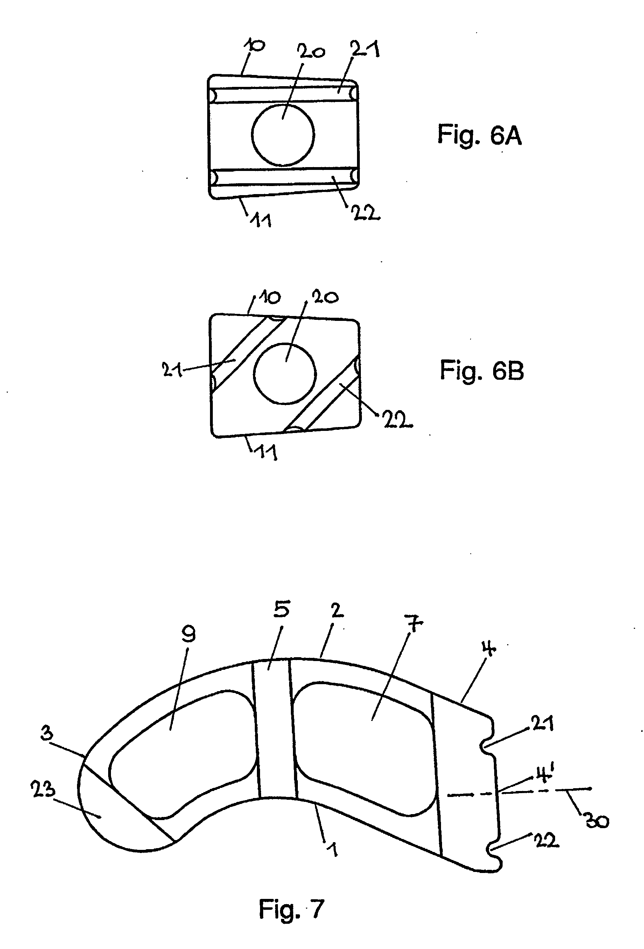

Fig. 6A und 6B zeigen je eine Seitenansicht des Heckteils mit verschieden um die Bohrung 20 angeordneten Führungselementen 21 und 22.

In Fig. 6A sind die Führungselemente in etwa parallel zu den Käfigflächen 10, 11 angeordnet, während sie in Fig. 6B einen Winkel von ca. 45° zu den Käfigflächen aufweisen. Dieser Winkel kann aber von 0 bis 90° einen beliebigen Wert annehmen. Die Führungselemente 21, 22 ihrerseits müssen allerdings nicht notwendigerweise parallel zueinander angeordnet sein; sie können auch eine V-förmige Anordnung aufweisen.

Als Materialien kommen Kunststoffe, carbonfaser-verstärkte Kunststoffe und Metalle, bzw. Metall-Legierungen in Frage. Bevorzugt werden Kunststoffe wie Polyetheretherketone (PEEK), Polyetherketonetheretherketone (PEKEEK) und Polysulfone (PS) eingesetzt, und besonders bevorzugt als Kompositwerkstoff carbonfaserverstärkte Komposite aus Polyetheretherketon (CFK/PEEK) und Polyetherketonetheretherketone (PEKEEK), die auch unter den Namen ULTRAPEK und OSTAPEK bekannt sind.

Als Metalle, bzw. Metall-Legierungen werden bevorzugt Titan und dessen Legierungen angewendet, wie z. B. die Titanlegierung Ti6-Al4-V gemäss ISO-Norm 5832-3.

Der metallische Käfig kann eine Hydroxyl-Apatit-Keramik (HAK)-Beschichtung oder eine Tri-Calcium-Phosphat (TCP)-Beschichtung aufweisen, wodurch die Langzeiteigenschaften des Implantats vorteilhaft beeinflusst werden.

Durch die gekrümmte Form des Käfigs weist dieser ein vorteilhaftes hohes Kippmoment M, das einem Umkippen des Käfigs wirkungsvoll entgegenwirkt. Im Vergleich mit dem Kippmoment eines quaderförmigen Käfigs mit gleichem mittigem Querschnitt, gleicher Länge und vergleichbarer Käfigstruktur hat sich gezeigt, dass die erfindungsgemässen Käfigstrukturen dieses um einen Faktor von mindestens 1,30 übertreffen. Für einen Käfig nach Fig. 1 liegt der Faktor bei 1,58.

Die Vorteile der erfindungsgemässen Käfigstruktur ergeben sich aus der doppelkeilförmigen Geometrie, die durch die beiden Lordosewinkel $\alpha 1$ und $\alpha 2$ bedingt ist und die sich der Anatomie im Zwischenwirbelbereich hervorragend anpasst.

Deshalb wird auch die Bezeichnung 'Double-Wedge-Shaped Cage', bzw. 'DWS Cage' für einen derartigen Käfig verwendet.

Die auf den Käfigflächen angebrachten Überhöhungen verhindern eine Migration des Käfigs nach erfolgtem Eingriff während dem Heilprozess wirkungsvoll.

Käfigstrukturen der beschriebenen Art zeichnen sich durch eine hohe Festigkeit aus, die trotz einem geringen Materialanteil zu erzielen ist. Dadurch wird die Bildung von Knochensubstanz stark beschleunigt.

Es hat sich gezeigt, dass sich diese Eigenschaft durch einen Cage Mass Index (CMI) beschrieben werden kann, der nach Formel (I) definiert ist

$$CMI = Volume\ of\ cage\ material\ /\ Volume\ of\ cage \qquad (I)$$

nämlich als Verhältniszahl des Käfigmaterialvolumens zum gesamten Käfigvolumen. Damit haben sich ergeben:

a) für CFK/PEEK, CFK/PEKEK, CFK/PS weniger als 0,25, vorzugsweise 0,22, und
b) für Titan, bzw. Ti-Legierungen weniger als 0,20, vorzugsweise 0,17,

wobei die Variationen bedingt durch die Käfiggrössen berücksichtigt sind und nur unwesentliche Unterschiede liefern.

Im weiteren wird das Verfahren zur Herstellung eines derartigen Käfigs beschrieben.

Dieses gliedert sich in vier Verfahrensschritte wie folgt:

1. Wasserstrahlschneiden

In einem ersten Schritt wird ein Rohling aus Käfigmaterial in einer ersten Richtung mittels eines Höchstdruck-Wasserstrahls bearbeitet. Dieses bekannte und wirtschaftliche Kaltschneideverfahren wird in der Regel mit einem Abrasivzusatz bei 3000 bar betrieben (U. W. Hunziker-Jost, Schweizer Präzisions-Fertigungstechnik, S. 81-86, C. Hanser Verlag, München (1991)).

Der Rohling ist so aufgespannt, dass der Wasserstrahl senkrecht auf die spätere Käfigfläche gerichtet ist. Dabei werden die Konturen der Seitenwände 1, 2, des Frontteils 3, des Heckteils 4, der mindestens einen Zwischenwand 5, 6, der mindestens zwei Hohlräume 7, 8, 9 und der Führungselemente 21, 22 mit hoher Präzision geschnitten. Die Schnittkanten weisen kaum Ausfransungen auf. Bei Materialdicken von 10 mm werden bei Konturenschnitten Schneidegeschwindigkeiten bis zu 100 mm/min für Metalle und bis zu 300 mm/min für Kompositwerkstoffe erzielt.

2. Fräsen

Der so aus dem Rohling getrennte Käfigrohling wird nun in einem zweiten Schritt neu eingespannt, und zwar in einer zur ersten im Wesentlichen senkrecht stehenden zweiten Richtung, wo der Käfigrohling mit einem Fräser weiterbearbeitet wird. Es werden die Käfigflächen 10, 11 entsprechend dem ersten Lordosewinkel $\alpha 1$, die tafelförmigen Überhöhungen 24, 25, 26, 27 entsprechend dem zweiten Lordosewinkel $\alpha 2$, die abgeschrägten Flächen 23, 23' des Frontteils 3, die mindestens eine Aussparung der Seitenwände 1 und die erste Bohrung 20 gefräst. Ebenfalls in diesem Schritt wird die Bohrung 20 mit dem Innengewinde versehen, das für die Instrumentenaufnahme vorgesehen ist. Kleinkalibrige Fräseinsätze gelangen dabei auf einem CNC-gesteuerten Fräsautomaten zum Einsatz.

Falls auf der äusseren Seitenwand 2 ebenfalls Aussparungen vorgesehen sind, muss der Käfigrohling noch einmal neu aufgespannt werden.

3. Marker anbringen

In einem dritten Schritt werden die Marker am Käfigrohling angebracht, die später während dem Eingriff und danach eine Beurteilung der Posititon des Käfigs mittels einem Bildverstärker ermöglichen. Zweite Bohrungen 31, 32, 33 für die Marker im Heckteil und des Markers im Frontteil werden angebracht, in welche die Marker als Kugeln und/oder Stifte aus Tantal eingefügt werden.

4. Endbearbeitung

In einem vierten Schritt erfolgen die letzten Operationen, die als Endbearbeitung zusammengefasst werden, nämlich das Trovalisieren, um die teilweise scharfen Kanten zu entgraten, bzw. zu runden. Danach folgt die Beschriftung des Käfigs, was z.B. mittels einer Laserbeschriftungsvorrichtung erfolgen kann. Anschliessend wird der Käfig einem Reinigungsprozess unterworfen, der z.B. eine mehrstufige Ultraschallreinigung einschliesst. Die Verpackung des Käfigs gehört ebenfalls in diese Endbearbeitung.

Wesentlicher Verfahrensschritt ist das Schneiden mit einem Wasserstrahl unter hohem Druck. Damit wurde ein vorteilhaftes Trennverfahren gewählt, welches sich als besonders wirtschaftlich erwiesen hat.

Die anschliessend beschriebenen Beispiele geben einen Einblick in die Vielfalt der Käfiggestaltung und deren Aufzählung ist keinesfalls abschliessend zu betrachten.

Fig. 7 zeigt als Ausführungsbeispiel einen Käfig mit einer Zwischenwand und einem schrägen Heckteil mit Aussparungen in Draufsicht.

Seitenwände 1, 2, Frontteil 3 mit Überhöhung und Abschrägung 23, Zwischenwand 5 mit Überhöhung, die Hohlräume 7, 9 entsprechen den in Fig. 1 beschriebenen Käfigelementen. Der Heckteil 4 mit Überhöhung bezüglich der Käfigfläche weist hier aber eine rautenförmige Ausbildung auf. Während die Führungselemente 21, 22, hier als Aussparungen ausgebildet, aber gleich wie in Fig. 1 auf der Heckteilfläche 4' angeordnet sind, zeigt die Richtung der Bohrung für die Instrumentenaufnahme an den Rand des Hohlraumes 7, was durch die Lage der Achse 30 für die Bohrung angedeutet ist. Die Bohrung ist mit einem Innengewinde M4 ausgestattet.

Die Käfigflächen bilden einen Lordosewinkel $\alpha 1$ von 3° in Richtung Frontteil - Heckteil und einen Lordosewinkel $\alpha 2$ von 2° in Richtung der Zentren für die Krümmungsradien der gekrümmten Seitenwände 1 und 2. So beträgt die Höhe der Zwischenwand mit Überhöhung aussenseitig 8,1 mm und innenseitig 7,8 mm. Die Seitenwand 1 weist zwei seitliche Aussparungen auf, die etwa in der Mitte der Hohlräume 7 und 9 liegen. Der innere Krümmungsradius R1 beträgt 11 mm, der äussere Krümmungsradius R2 19 mm, wobei die entsprechenden Zentren um 1,1 mm versetzt waren. Der Käfig wurde aus CFK/PEEK gefertigt, wobei im ersten Verfahrensschritt eine Wasserstrahlschneidanlage BYJET (Bystronic Laser AG, CH-3362 Niederönz) eingesetzt wurde.

Als weiteres Ausführungsbeispiel wurde die Käfigstruktur gemäss Fig. 6 aus einer Titanlegierung Ti6-Al4-V gemäss ISO-Norm 5832-3 gefertigt, wobei ebenfalls im ersten Verfahrensschritt eine Wasserstrahlschneidanlage BYJET zur Anwendung gelangte.

**Patentansprüche**

1.  Käfig für ein käfigartiges Zwischenwirbelimplantat, umfassend einen in Hohlräume unterteilten Körper, wobei der Käfig (100) von einer konkavgekrümmten Seitenwand (1), von einer konvex-gekrümmten Seitenwand (2), einem Frontteil (3) und einem Heckteil (4) gebildet wird, wobei die innere Seitenwand (1) einem ersten inneren Krümmungsradius (R1) und die äussere Seitenwand (2) einem zweiten äusseren Krümmungsradius (R2) zugeordnet ist, dass die Seitenwände mit Frontteil und Heckteil verbunden sind, dass die Seitenwände (1, 2) mit mindestens einer Zwischenwand (5, 6) verbunden sind, die im Wesentlichen senkrecht zu den Seitenwänden steht, wodurch der Käfig (100) mindestens 2 Hohlräume (7, 8, 9) aufweist und dass Seitenwände (1, 2), Frontteil (3), Heckteil (4) und die mindestens eine Zwischenwand (5, 6) eine obere und eine untere Käfigfläche (10, 11) bilden, **gekennzeichnet dadurch, dass** die obere und die untere Käfigfläche einen ersten Lordosewinkel ($\alpha$1) in Richtung Frontteil-Heckteil und einen zweiten Lordosewinkel ($\alpha$2) senkrecht dazu bilden, indem sich die Käfigflächen (10, 11) ausserhalb des Käfigs schneiden.

2. Käfig nach Anspruch 1, **gekennzeichnet dadurch, dass** die innere Seitenwand (1) eine geringere Höhe als die äussere Seitenwand (2) oder die äussere Seitenwand (2) eine geringere Höhe als die innere Seitenwand (1) aufweist, wodurch ein zweiter Lordosewinkel ($\alpha$2) gebildet wird.

3. Käfig nach Anspruch 1 oder 2, **gekennzeichnet dadurch, dass** der Frontteil (3) und der Heckteil (4) im Wesentlichen den gleichen zweiten Lordosewinkel ($\alpha$2) aufweisen.

4. Käfig nach einem der Ansprüche 1 - 3, **gekennzeichnet dadurch, dass** der Lordosewinkel ($\alpha$1) 2 - 8°, vorzugsweise 3, 5 oder 7° beträgt.

5. Käfig nach einem der Ansprüche 1 - 4, **gekennzeichnet dadurch, dass** der Lordosewinkel ($\alpha$2) 0,1 - 4°, vorzugsweise 2° beträgt, und im Grenzfall identisch null wird.

6. Käfig nach einem der Ansprüche 1 - 5, **gekennzeichnet dadurch, dass** der erste innere Krümmungsradius (R1) und der zweite äussere Krümmungsradius (R2) im Wesentlichen die gleichen Zentren, aber vorzugsweise versetzt angeordnete Zentren aufweisen.

7. Käfig nach einem der Ansprüche 1 - 6, **gekennzeichnet dadurch, dass** der innere Krümmungsradius (R1) 15 - 23 mm, vorzugsweise 22 mm, und der äussere Krümmungsradius (R2) 18 - 26 mm, vorzugsweise 19, 21 oder 23 mm beträgt.

8. Käfig nach einem der Ansprüche 1 - 7, **gekennzeichnet dadurch, dass** die innere Seitenwand (1) mindestens eine Aussparung (13, 14, 15) aufweist.

9. Käfig nach einem der Ansprüche 1 - 8, **gekennzeichnet dadurch, dass** die äussere Seitenwand (2) mindestens eine Aussparung aufweist.

10. Käfig nach einem der Ansprüche 1 - 9, **gekennzeichnet dadurch, dass** der Frontteil (3) abgerundet ausgebildet ist und mindestens eine angeschrägte Fläche (23) aufweist.

11. Käfig nach einem der Ansprüche 1 - 10, **gekennzeichnet dadurch, dass** der Heckteil (4) in dessen Mitte eine Bohrung (20) mit Innengewinde aufweist, die von zwei im Wesentlichen parallel oder V-förmig verlaufenden Führungselementen (21, 22) umgeben ist.

12. Käfig nach Anspruch 11, **gekennzeichnet dadurch, dass** die Führungselemente (21, 22) in etwa senkrecht oder in etwa parallel oder in einem Winkel von 0 bis 90°, vorzugsweise 45°, zu den Käfigflächen um die Bohrung (20) angeordnet sind.

13. Käfig nach Anspruch 11 oder 12, **gekennzeichnet dadurch, dass** die Führungselemente (21, 22) als Rippen oder Aussparungen ausgebildet sind.

14. Käfig nach einem der Ansprüche 1 - 13, **gekennzeichnet dadurch, dass** im Frontteil (3) und im Heckteil (4) Bohrungen (31, 32, 33) für Marker aus einem Metall hoher Dichte als Kugeln und/oder Stifte vorgesehen sind.

15. Käfig nach Anspruch 14, **gekennzeichnet dadurch, dass** als Metall für die Marker Tantal vorgesehen ist.

16. Käfig nach Anspruch 14 oder 15, **gekennzeichnet dadurch, dass** die Marker als Stifte in etwa senkrecht oder parallel zur Bohrung (20) angeordnet sind.

17. Käfig nach einem der Ansprüche 1 - 16, **gekennzeichnet dadurch, dass** die Käfigflächen (10, 11) tafelförmige Überhöhungen (24, 25, 26, 27) aufweisen, die im Wesentlichen parallel zu den Käfigflächen verlaufen.

18. Käfig nach einem der Ansprüche 1 - 17, **gekennzeichnet dadurch, dass** er ein in Richtung des Zentrums des inneren Krümmungsradius (R1) gerichtetes Kippmoment (M) aufweist, das verglichen mit jenem eines quaderförmigen Käfigs gleichen Querschnitts, gleicher Länge und vergleichbarer Käfigstruktur dieses um einen Faktor von mindestens 1,30 übertrifft.

19. Käfig nach einem der Ansprüche 1 - 18, **gekennzeichnet dadurch, dass** er aus Kunststoff, vorzugsweise aus Polyetheretherketon (PEEK), Polyetherketonetheretherketon (PEKEEK) oder aus Polysulfon (PS), oder einem Kompositmaterial besteht, vorzugsweise aus carbonfaserverstärktem Komposit aus CFK/PEEK und CFK/-PEKEEK.

20. Käfig nach einem der Ansprüche 1 - 18, **gekennzeichnet dadurch, dass** er aus Titan oder einer Ti-Legierung besteht.

21. Käfig nach Anspruch 20, **gekennzeichnet dadurch, dass** er eine Hydroxyl-Apatit-Keramik (HAK)-Beschichtung oder eine Tri-Calcium-Phosphat (TCP)-Beschichtung aufweist.

22. Käfig nach einem der Ansprüche 1-21, **gekennzeichnet dadurch, dass** er einen Cage Mass Index (CMI) aufweist, der nach Formel (I)

$$CMI = \text{Volume of cage material} / \text{Volume of cage} \qquad (I)$$

für CFK/PEEK, CFK/PEKEEK und PS weniger als 0,25, vorzugsweise 0,22, und für Titan, bzw. Ti-Legierungen weniger als 0,20, vorzugsweise 0,17, beträgt.

23. Verfahren zur Herstellung eines Käfigs nach einem der Ansprüche 1 - 22, **gekennzeichnet dadurch, dass** in einem ersten Schritt ein Rohling aus Käfigmaterial in einer ersten Richtung mittels eines Höchstdruck-Wasserstrahls bearbeitet wird, wobei die Konturen der Seitenwände (1, 2), des Frontteils (3), des Heckteils (4), der mindestens einen Zwischenwand (5, 6), der mindestens zwei Hohlräume (7, 8, 9) und der Führungselemente (21, 22) entstehen, dass in einem zweiten Schritt in einer zur ersten im Wesentlichen senkrecht stehenden zweiten Richtung der Rohling gefräst wird, dass in einem dritten Schritt die Marker angebracht werden und dass in einem vierten Schritt die Endbearbeitung erfolgt.

24. Verfahren nach Anspruch 23, **gekennzeichnet dadurch, dass** im zweiten Schritt die Käfigflächen (10, 11) entsprechend dem ersten Lordosewinkel ($\alpha$1), die tafelförmige Überhöhungen (24, 25, 26, 27) entsprechend dem zweiten Lordosewinkel ($\alpha$2), die abgeschrägten Flächen (23, 23') des Frontteils (3), die mindestens eine Aussparung (13, 14, 15) der Seitenwand (1) und die erste Bohrung (20) fräst werden.

25. Verfahren nach Anspruch 24, **gekennzeichnet dadurch, dass** die erste Bohrung (20) mit einem Innengewinde versehen wird.

26. Verfahren nach einem der Ansprüche 22 - 25, **gekennzeichnet dadurch, dass** im dritten Schritt zweite Bohrungen (31, 32, 33) für die Marker angebracht und die Marker eingefügt werden.

27. Verfahren nach einem der Ansprüche 22 - 26, **gekennzeichnet dadurch, dass** die Endbearbeitung des bearbeiteten Rohlings das Trovalisieren, Beschriften, die Reinigung und die Verpackung umfasst.

**Claims**

1. Cage for a cage-like intervertebral implant, comprising a member divided into cavities, the cage (100) being constituted by a concave-curved sidewall (1), a convex-curved sidewall (2), a front portion (3), and a rear portion (4), a first, inner radius of curvature (R1) being allocated to the inner sidewall (1), and a second, outer radius of curvature (R2) being allocated to the outer sidewall (2); the sidewalls are connected to the front portion and the rear portion; the sidewalls (1, 2) are connected to at least one partition (5, 6) substantially perpendicular to the sidewalls, whereby the cage (100) has at least two cavities (7, 8, 9); and the sidewalls (1, 2), front portion (3), rear portion (4), and the at least one partition (5, 6) form an upper and a lower cage surface (10, 11), wherein the upper and the lower cage surface (10, 11) are forming a lordosis angle ($\alpha$1) in the front portion - rear portion direction, and a second lordosis angle ($\alpha$2) perpendicular thereto, in that the cage surfaces (10, 11) intersect outside the cage.

2. Cage according to claim 1, wherein the inner sidewall (1) has a smaller height than the outer sidewall (2), or the outer sidewall (2) has a smaller height than the inner sidewall (1), whereby a second lordosis angle ($\alpha$2) is formed.

3. Cage according to claim 1 or 2, wherein the front portion (3) and the rear portion (4) have a substantially equal second lordosis angle ($\alpha$2).

4. Cage according to one of claims 1-3, wherein the lordosis angle ($\alpha$1) is 2-8°, preferably 3, 5, or 7°.

5. Cage according to one of claims 1-4, wherein the lordosis angle ($\alpha$2) is 0.1-4°, preferably 2°, and in the limiting case is identically zero.

6. Cage according to one of claims 1-5, wherein the first, inner radius of curvature (R1) and the second, outer radius of curvature (R2) have substantially the same centers, but preferably centers arranged offset.

7. Cage according to one of claims 1-6, wherein the inner radius of curvature (R1) is 15-23 mm, preferably 22 mm, and the outer radius of curvature (R2) is 18-26 mm, preferably 19, 21 or 23 mm.

8. Cage according to one of claims 1-7, wherein the inner sidewall (1) has at least one opening (13, 14, 15).

9. Cage according to one of claims 1-8, wherein the outer sidewall (2) has at least one opening.

10. Cage according to one of claims 1-9, wherein the front portion (3) is rounded and has at least one beveled surface (23).

11. Cage according to one of claims 1-10, wherein the rear portion (4) has in its middle a hole (20) with an internal thread, which is surrounded by two guide elements (21, 22) which run substantially parallel or in a V-shape.

12. Cage according to claim 11, wherein the guide elements (21, 22) are arranged about perpendicular, or about parallel, or at an angle of 0° to 90°, preferably 45°, to the cage surfaces around the hole (20).

13. Cage according to claim 11 or 12, wherein the guide elements (21, 22) are constituted as ribs or recesses.

14. Cage according to one of claims 1-13, wherein holes (31, 32, 33) are provided in the front portion (3) and in the rear portion (4) for markers of a metal of high density as balls and/or pins.

15. Cage according to claim 14, wherein tantalum is provided as the metal for the markers.

16. Cage according to claim 14 or 15, wherein the markers are arranged as pins about perpendicular or parallel to the hole (20).

17. Cage according to one of claims 1-16, wherein the cage surfaces (10, 11) have tabular raised portions (24, 25, 26, 27) which run substantially parallel to the cage surfaces.

18. Cage according to one of claims 1-17, wherein it has a tilting moment (M) directed in the direction of the center of the inner radius of curvature (R1) and, in comparison with the tilting moment of a cuboidal cage of the same cross section, same length and comparable cage structure, exceeding this tilting moment by a factor of at least 1.30.

19. Cage according to one of claims 1-18, wherein it consists of plastic, preferably of polyether ether ketone (PEEK), polyether ketone ether ether ketone (PEKEEK), or of polysulfone (PS), or of a composite material, preferably carbon fiber reinforced composites of CFK/PEEK and CFK/PEKEEK.

20. Cage according to one of claims 1-18, wherein it consists of titanium or a Ti alloy.

21. Cage according to claim 20, wherein it has a hydroxyapatite ceramic (HAK) coating or a tricalcium phosphate (TCP) coating.

22. Cage according to one of claims 1-21, wherein it has a Cage Mass Index (CMI) according to Equation (1)

$$CMI = \text{Volume of cage material} / \text{Volume of cage} \tag{1}$$

which for CFK/PEEK, CFK/PEKEEK, and PS is less than 0.25, preferably 0.22, and for titanium or Ti alloys, less than 0.20, preferably 0.17.

23. Process for the production of a cage according to one of claims 1-22, wherein in a first step a blank of cage material is machined in a first direction by means of an ultrahigh pressure water jet, the contours of the sidewalls (1, 2), of the front portion (3), the rear portion (4), the at least one sidewall (5, 6), the at least two cavities (7, 8, 9), and the guide elements (21, 22) are arising; and in a second step the blank is milled in a direction substantially perpendicular to the first direction; and in a third step, the markers are installed; and finishing follows in a fourth step.

24. Process according to claim 23, wherein, in the second step, the cage surfaces (10, 11) are milled according to the first lordosis angle ($\alpha$1), the tabular raised portions (24, 25, 26, 27) are milled corresponding to the second lordosis angle ($\alpha$2); and the beveled surfaces (23, 23') of the front portion (3), the at least one opening (13, 14, 15) of the sidewall (1), and the first hole (20) are milled.

25. Process according to claim 24, wherein the first hole (20) is provided with an internal thread.

26. Process according to one of claims 22-25, wherein, in the third step, second holes (31, 32, 33) are formed for the markers, and the markers are inserted.

27. Process according to one of claims 22-26, wherein the final processing of the machine blank includes trovalization, marking, cleaning, and packaging.

**Revendications**

1. Cage pour un implant intervertebral de type cage, comprenant un corps subdivisé en cavités où la cage (100) est constituée d'une paroi latérale concave (1), d'une paroi latérale convexe (2), d'une partie frontale (3) et d'une partie dorsale (4), considérant que la paroi latérale intérieure (1) comporte un premier rayon de courbure (R1) et la paroi latérale extérieure (2) comporte un second rayon de courbure (R2), que les parois latérales sont reliées à la partie frontale et à la partie dorsale, que les parois latérales (1,2) sont reliées avec au moins une paroi intermédiaire (5, 6), laquelle est pour l'essentiel disposée verticalement par rapport aux parois latérales, où la cage (100) comporte au moins 2 cavités (7, 8, 9) et que les parois latérales (1, 2), la partie frontale (3) et la partie dorsale (4) et au moins une paroi intermédiaire (5, 6) forment une surface de cage supérieure et inférieure (10,11), **caractérisée par le fait que** la surface de cage supérieure et inférieure forment un premier angle de lordose ($\alpha$1) en direction de la partie frontale-dorsale et un second angle de lordose ($\alpha$2) perpendiculaire à celui, en tenant compte que les surfaces de cage (10, 11) se coupent à l'extérieur de la cage.

2. Cage selon revendication 1, **caractérisée par le fait qu'**une paroi latérale intérieure (1) possède une hauteur inférieure à la paroi latérale extérieure (2) ou que la paroi latérale extérieure (2) possède une hauteur inférieure à la paroi latérale intérieure (1), compte tenu qu'un second angle de lordose ($\alpha$2) est formé.

3. Cage selon revendications 1 ou 2, **caractérisée par le fait que** la partie frontale (3) et la partie dorsale (4) présentent pour l'essentiel le même angle de lordose ($\alpha$2).

4. Cage selon revendications 1 - 3, **caractérisée par le fait que** l'angle de lordose ($\alpha$1) soit de 2 - 8°, de préférence 3, 5 ou 7°.

5. Cage selon revendications 1 - 4, **caractérisée par le fait que** l'angle de lordose ($\alpha$2) soit de 0,1 - 4°, de préférence 2° et que dans un cas limite il soit identique à zéro.

6. Cage selon revendications 1 - 5, **caractérisée par le fait que** le premier rayon de courbure intérieur (R1) et le second rayon de courbure extérieur (R2) possède pour l'essentiel les mêmes centres, mais comportent de préférence des centres disposés de façon décalée.

7. Cage selon revendications 1 - 6, **caractérisée par le fait que** le premier rayon de courbure intérieur (R1) soit de 15 - 23 mm, de préférence 22 mm et le second rayon de courbure extérieur (R2) soit de 18 - 26 mm, de préférence de 19, 21 ou 23 mm.

8. Cage selon revendications 1 - 7, **caractérisée par le fait que** la paroi latérale intérieure (1) comporte au moins un évidement (13, 14, 15).

9. Cage selon revendications 1 - 8, **caractérisée par le fait que** la paroi latérale extérieure (2) comporte au moins un évidement.

10. Cage selon revendications 1 - 9, **caractérisée par le fait que** la partie frontale (3) est configurée de façon arrondie et comporte au moins une surface biaisée (23).

11. Cage selon revendications 1 - 10, **caractérisée par le fait que** la partie dorsale (4) comporte en son centre un perçage (20) avec filetage intérieur, flanqué de deux éléments de guidage (21, 22) disposés essentiellement en parallèle ou en forme de V.

12. Cage selon revendication 11, **caractérisée par le fait que** les éléments de guidage (21, 22) sont disposés environ en perpendiculaire ou environ en parallèle ou formant un angle de 0° à 90°, de préférence 45°, par rapport aux surfaces de cage autour du perçage.

13. Cage selon revendication 11 ou 12, **caractérisée par le fait que** les éléments de guidage (21, 22) sont conçus comme nervure ou évidement.

14. Cage selon revendications 1 - 13, **caractérisée par le fait que** dans la partie frontale (3) et dans la partie dorsale (4) sont prévus des perçages (31, 32, 33) pour des traceurs d'un métal de haute densité en forme de sphères et/ou de tiges.

15. Cage selon revendication 14, **caractérisée par le fait que** le métal prévu pour les traceurs est du tantale.

16. Cage selon revendication 1 ou 15, **caractérisée par le fait que** les traceurs comme tiges sont disposés environ en perpendiculaire ou parallèlement au perçage (20).

17. Cage selon revendications 1 - 16, **caractérisée par le fait que** les surfaces de cage (10, 11) comportent des surélévations en forme de table (24, 25, 26, 27), disposées pour l'essentiel en parallèle avec les surfaces de cage.

18. Cage selon revendications 1 - 17, **caractérisée par le fait qu'**elle présente un couple de pivotement en direction du centre du rayon de courbure intérieur (R1) qui surpasse d'un facteur d'au moins 1,30 celui, par comparaison, d'une cage de forme quadratique de même section, de même longueur et de structure de cage comparable.

19. Cage selon revendications 1 - 18, **caractérisée par le fait qu'**elle soit en matière synthétique, de préférence en polyetherethercétone (PEEK), polyethercétoneetherethercétone (PEKEEK) ou en polysulfon (PS) ou un matériel composite, de préférence un composite renforcé de fibres de carbone en CFK/PEEK et CFK/PEKEEK.

20. Cage selon revendications 1 - 18, **caractérisée par le fait qu'**elle soit composée de titane ou d'un alliage de titane.

21. Cage selon revendication 20, **caractérisée par le fait qu'**elle comporte un revêtement d'hydroxyl-apatite-céramique (HAK) ou de phosphate tri-calcium (TCP).

22. Cage selon revendications 1 - 21, **caractérisée par le fait qu'**elle comporte un index de mesure de cage (CMI), déterminé selon la formule (I)

CMI = volume du matériau de cage / volume de cage

valant pour le CFK/PEEK, CFK/PEKEEK et PS moins de 0,25, de préférence 0,22 et pour le titane moins de 0,20, de préférence 0,17.

**23.** Procédé pour la réalisation d'une cage selon revendications 1 - 22 **caractérisé par le fait que** dans une première phase une pièce brute dans le matériau de la cage est façonné dans une première direction au moyen d'un jet d'eau sous pression extrême, procédé au cours duquel les contours des parois latérales (1, 2), de la partie frontale (3), de la partie dorsale (4), d'au moins une paroi intermédiaire (5, 6), d'au moins 2 évidements (7, 8, 9) et des éléments de guidage (21, 22) sont réalisés, que dans une deuxième phase l'ébauche est fraisée dans une deuxième direction pour l'essentiel en perpendiculaire à la première, que dans une troisième phase les traceurs soient rapportés et que dans une quatrième phase ait lieu l'usinage final.

**24.** Procédé selon revendication 23, **caractérisé par le fait que** dans une deuxième phase soient fraisées les surfaces de cage (10, 11) de façon à correspondre au premier angle de lordose ($\alpha 1$), les surélévations en forme de table (24, 25, 26, 27) de façon à correspondre à l'angle de lordose ($\alpha 2$), les surfaces biaisées (23, 23') de la partie frontale (3), l'au moins un évidement (13, 14, 15) de la paroi latérale (1) et le premier perçage (20).

**25.** Procédé selon revendication 24, **caractérisé par le fait que** le premier perçage (20) soit pourvu d'un filetage intérieur.

**26.** Procédé selon revendications 22 - 25, **caractérisé par le fait que** dans une troisième phase soient réalisés des seconds perçages (31, 32, 33) destinés aux traceurs et que les traceurs y soient introduits.

**27.** Procédé selon revendications 22 - 26, **caractérisée par le fait que** l'usinage final de la pièce brute en travail, comprend la trovalisation, l'inscription, le nettoyage et l'emballage.

Fig. 1

Fig. 2

A - A'

Fig. 3A

$\frac{\alpha 2}{2}$

B - B'

Fig. 3B

C - C'

Fig. 3C

D - D'

Fig. 3D

E - E'    Fig. 4A

$\dfrac{\alpha 2}{2}$

F - F'    Fig. 4B

G - G'    Fig. 4C

Fig. 5

EP 1 274 375 B1

Fig. 6A

Fig. 6B

Fig. 7